# EUROPÄISCHE PATENTSCHRIFT

(11) **EP 1 102 733 B1**
(45) Veröffentlichungstag und Bekanntmachung des Hinweises auf die Patenterteilung: **07.04.2004**
(21) Anmeldenummer: 99942819.6
(22) Anmeldetag: 09.08.1999
(51) Int. Cl.: C07B 41/00, C07D 301/12, C07D 301/19, B01J 8/04

(54) **VERFAHREN ZUR UMSETZUNG EINER ORGANISCHEN VERBINDUNG MIT EINEM HYDROPEROXID**
METHOD FOR REACTING AN ORGANIC COMPOUND WITH A HYDROPEROXIDE
PROCEDE PERMETTANT DE TRANSFORMER UN COMPOSE ORGANIQUE AU MOYEN D'UN HYDROPEROXYDE

(30) Priorität: 07.08.1998 DE 19835907
(43) Veröffentlichungstag der Anmeldung: 30.05.2001
(73) Patentinhaber: BASF AKTIENGESELLSCHAFT, 67056 Ludwigshafen (DE)
(72) Erfinder: BASSLER, Peter, D-68519 Viernheim (DE); HARDER, Wolfgang, D-69469 Weinheim (DE); RESCH, Peter, D-67310 Hettenleidelheim (DE); RIEBER, Norbert, D-68259 Mannheim (DE); RUPPEL, Wilhelm, D-67227 Frankenthal (DE); TELES, Joaquim, Henrique, D-67122 Altrip (DE); WALCH, Andreas, D-74193 Schwaigern (DE); WENZEL, Anne, D-76676 Graben-Neudorf (DE); ZEHNER, Peter, D-67071 Ludwigshafen (DE)
(74) Vertreter: Isenbruck, Günter, Dr.
(86) Internationale Anmeldenummer: PCT/EP1999/005740
(87) Internationale Veröffentlichungsnummer: WO 2000/007965

(56) Entgegenhaltungen:
- EP-A- 0 031 537
- EP-A- 0 568 336
- EP-A- 0 659 473
- US-A- 4 977 285
- US-A- 5 274 138
- US-A- 5 349 072
- US-A- 5 374 747
- US-A- 5 384 418
- US-A- 5 463 090

## Beschreibung

Die vorliegende Erfindung betrifft ein Verfahren zur Umsetzung einer organischen Verbindung mit einem Hydroperoxid, wobei im Laufe des Verfahrens Hydroperoxid abgetrennt wird und erneut mit der organischen Verbindung umgesetzt wird. Ebenso betrifft die vorliegende Erfindung eine Vorrichtung zur Umsetzung einer organischen Verbindung mit einem Hydroperoxid.

Umsetzungen von organischen Verbindungen mit Hydroperoxiden, d.h. mit Verbindungen der allgemeinen Formel ROOH, werden in den gängigen Verfahren des Standes der Technik im Regelfall einstufig durchgeführt.

Der Begriff "einstufig" bezieht sich in diesem Zusammenhang auf das Hydroperoxid-Edukt und bedeutet, daß während des gesamten Verfahrens nur in einem einzigen Schritt Hydroperoxid der umzusetzenden organischen Verbindung zugegeben wird.

Die US-A-5,262,550 beschreibt beispielsweise ein Verfahren zur Epoxidierung von Alkenen, in dem in einer Stufe Alken mit Wasserstoffperoxid oder einem Wasserstoffperoxid-Precursor zum entsprechenden Alkenoxid umgesetzt wird.

Die US-A-4,883,260 offenbart ein Verfahren, in dem Alken mit Wasserstoffperoxid in einer Stufe im Stahlautoklaven bzw. im Glasautoklaven umgesetzt wird.

In S.-H. Wang, Process Economics Program, Report 2E, S. 6-1 bis 6-27, SRI International (1994) ist beispielsweise ein Verfahren beschrieben, in dem in einer Stufe eine ca. 17 Gew.-%ige Ethylbenzolhydroperoxidlösung mit Propen an einem homogenen Mo-Katalysator umgesetzt wird. Insgesamt werden bei diesem Verfahren pro Mol Hydroperoxid 7,2 mol Propen eingesetzt.

Die gleiche Schrift offenbart auf den Seiten 6-28 bis 6-47 ein Verfahren, in dem in einer Stufe eine ca. 20 Gew.-%ige Ethylbenzolhydroperoxidlösung mit Propen an einem heterogenen Ti/SiO₂-Katalysator umgesetzt wird, wobei das Alken epoxidiert wird. Pro Mol Hydroperoxid werden hierbei 16,7 mol Propen eingesetzt.

Ebenfalls in dieser Schrift wird auf den Seiten 5-1 bis 5-21 ein Verfahren offenbart, in dem in einer Stufe eine ca. 40 Gew.-%ige tert-Butylhydroperoxidlösung mit Propen an einem homogenen Mo-Katalysator umgesetzt wird, wobei das Alken epoxidiert wird. Pro Mol Hydroperoxid werden hierbei 3,7 mol Propen eingesetzt.

Die gleiche Schrift offenbart auf den Seiten 5-22 bis 5-43 ein Verfahren, in dem in zwei direkt aufeinanderfolgenden Stufen eine ca. 72 Gew.-%ige tert-Butylhydroperoxidlösung mit Propen und einem homogenen Mo-Katalysator umgesetzt wird, wobei das Alken epoxidiert wird. Dabei werden pro Mol Hydroperoxid 1,2 Mol Propen eingesetzt.

Ein Nachteil dieser Verfahren ist darin zu sehen, daß entweder mit einem relativ hohen Überschuß an der umzusetzenden organischen Verbindung oder mit einem sehr konzentrierten Hydroperoxid gearbeitet werden muß, um optimale Selektivitäten zu erreichen.

Ferner ist aus der EP 0 568 338 ein Verfahren zur Epoxidierung von Olefinen in Gegenwart eines Katalysators aus Silicalit, der Titan enthält, bekannt. Nicht umgesetztes Olefin und Wasserstoffperoxid können bei diesem Verfahren abgetrennt und der Reaktion wieder zugeführt werden.

Aus der US 5,349,072 ist ein Verfahren zur Herstellung von Propylenoxid aus Propylen und t-Butylhydroperoxid als Oxidationsmittel bekannt, wobei die Umsetzung durch katalytische Oxidation in homogener Phase durchgeführt wird.

Auch die US 5,274,138 offenbart ein Verfahren zur katalytischen Epoxidation von Propylen mit t-Butylhydroperoxid in homogener Phase.

Eine Aufgabe der vorliegenden Erfindung war es daher, ein Verfahren bereitzustellen, das es erlaubt, den Überschuß an umzusetzender organischer Verbindung so gering wie möglich zu halten und ein relativ niedrig konzentriertes Hydroperoxid einzusetzen.

Daher betrifft die vorliegende Erfindung ein Verfahren zur Umsetzung einer organischen Verbindung mit einem Hydroperoxid, das mindestens die folgenden Stufen (i) bis (iii) umfaßt:
(i) Umsetzung des Hydroperoxides mit der organischen Verbindung unter Erhalt einer Mischung, umfassend die umgesetzte organische Verbindung und nicht umgesetztes Hydroperoxid,
(ii) Abtrennung des nicht umgesetzten Hydroperoxides aus der aus Stufe (i) resultierenden Mischung,
(iii) Umsetzung des abgetrennten Hydroperoxides aus Stufe (ii) mit der organischen Verbindung,
dadurch gekennzeichnet, dass als Hydroperoxid Wasserstoffperoxid verwendet wird, die organische Verbindung bei der Umsetzung mit einem heterogenen Katalysator in Kontakt gebracht wird und die Umsetzungen in den Stufen (i) und (iii) in zwei getrennten Festbettreaktoren durchgeführt werden.

Demgemäß findet die Umsetzung der organischen Verbindung mit dem Hydroperoxid in mindestens zwei Stufen (i) und (iii) statt. Ebenso ist es denkbar, daß die Umsetzung der organischen Verbindung mit dem Hydroperoxid in mehr als zwei Stufen stattfindet. Je nach Anzahl der Stufen, in denen die Umsetzung stattfindet, ist es selbstverständlich auch denkbar, daß mehr als eine Stufe durchlaufen wird, in der das eingesetzte Hydroperoxid abgetrennt wird.

Als Beispiel sei z.B. ein Verfahren genannt, in dem die Umsetzung der organischen Verbindung mit dem Hydroperoxid in den Stufen (i), (iii) und (v), die Abtrennung des Hydroperoxids in den Stufen (ii) und (iv) stattfindet.

Im allgemeinen werden zwei bis fünf Stufen durchlaufen, in denen die organische Verbindung mit dem Hydroperoxid umgesetzt wird. Eine Ausführungsform des Verfahrens weist die folgenden Stufen (i) bis (ix) auf:
(i) Umsetzung des Hydroperoxides mit der organischen Verbindung unter Erhalt einer Mischung M_{I},
(ii) Abtrennung des Hydroperoxides aus der aus Stufe (i) resultierenden Mischung M_{I},
(iii) Umsetzung des abgetrennten Hydroperoxides aus Stufe (ü) mit der organischen Verbindung unter Erhalt einer Mischung M_{II},
(iv) Abtrennung des Hydroperoxides aus der aus Stufe (iii) resultierenden Mischung M_{II},
(v) Umsetzung des abgetrennten Hydroperoxides aus Stufe (iv) mit der organischen Verbindung unter Erhalt einer Mischung M_{III},
(vi) Abtrennung des Hydroperoxides aus der aus Stufe (v) resultierenden Mischung M_{III},
(vii) Umsetzung des abgetrennten Hydroperoxides aus Stufe (vi) mit der organischen Verbindung unter Erhalt einer Mischung M_{IV},
(viii) Abtrennung des Hydroperoxides aus der aus Stufe (vii) resultierenden Mischung M_{IV},
(ix) Umsetzung des abgetrennten Hydroperoxides aus Stufe (viii) mit der organischen Verbindung.

Bevorzugt sind zwei bis vier Stufen, in denen die organische Verbindung mit Hydroperoxid umgesetzt wird, besonders bevorzugt zwei bis drei Stufen. Vorzugsweise findet die Umsetzung der organischen Verbindung mit dem Hydroperoxid in zwei Stufen statt.

Die Abtrennung des Hydroperoxids in den oben genannten Abtrennstufen (ii), (iv), (vi) und (viii) kann nach allen gängigen Verfahren gemäß dem Stand der Technik durchgeführt werden. Dabei können in unterschiedlichen Abtrennstufen auch unterschiedliche Abtrennmethoden eingesetzt werden.

Vorzugsweise erfolgt die Abtrennung des Hydroperoxides in den Abtrennstufen destillativ. Je nach den Anforderungen des Verfahrens ist dabei eine Abtrennung in einer oder mehreren Destillationskolonnen möglich. Vorzugsweise wird in einer Abtrennstufe zur Abtrennung des Hydroperoxides eine Destillationskolonne verwendet.

Es ist denkbar, für jede Stufe, in der das Hydroperoxid abgetrennt wird, eine eigene Abtrennvorrichtung Aᵢ vorzusehen. Ebenso ist es möglich, bei entsprechender Reaktionsführung und bei mehreren Abtrennstufen die Abtrennungen in einer einzigen Abtrennvorrichtung durchzuführen.

Sind mehrere Abtrennstufen vorgesehen, ist es auch möglich, durch geeignete Reaktionsführung jeweils zwei oder auch mehr Abtrennstüfen in jeweils einer Abtrennvorrichtung durchzuführen. Ganz allgemein ist es demgemäß möglich, für n Abtrennstufen insgesamt m Abtrennvorrichtungen vorzusehen, wobei 1 ≤ m ≤ n.

Sollte im Anschluß an die letzte Stufe, in der eine Umsetzung der organischen Verbindung mit dem Hydroperoxid stattfindet, noch eine weitere Abtrennung des Hydroperoxids gewünscht sein, beispielsweise, um eventuell restliches Hydroperoxid zu recyclen, so ist dies selbstverständlich ebenfalls möglich.

Aus der Mischung, die aus einer Umsetzungsstufe, in der die organische Verbindung mit dem Hydroperoxid umgesetzt wird, resultiert, kann in einer Abtrennvorrichtung neben dem Hydroperoxid auch die umgesetzte organische Verbindung abgetrennt werden. Natürlich ist es auch möglich, nach Abtrennung des Hydroperoxids das verbleibende Reaktionsgut in eine weitere, speziell zu diesem Zweck vorgesehene Abtrennvorrichtung zu überführen und dort aus dem Reaktionsgut die umgesetzte organische Verbindung abzutrennen.

In beiden Fällen ist es beispielsweise möglich, die umgesetzte organische Verbindung in den n Abtrennvorrichtungen zu sammeln und nach Beendigung der Umsetzungen der organischen Verbindung mit dem Hydroperoxid abzutrennen.

Bevorzugt wird die umgesetzte organische Verbindung jedoch in der jeweiligen Abtrennvorrichtung neben dem Hydroperoxid abgetrennt. Bei einer destillativen Abtrennung ist es beispielsweise möglich, die umgesetzte organische Verbindung über Kopf der Mischung zu entnehmen, und im Seitenabzug das Hydroperoxid aus der Mischung abzutrennen.

Es ist natürlich ebenfalls möglich, bei Verwendung einer Destillationsanlage als Abtrenneinrichtung das Hydroperoxid nicht über Seitenabzug, sondern über Sumpf aus der Mischung abzutrennen.

Erfolgt die Abtrennung des Hydroperoxids und/oder der umgesetzten organischen Verbindung in einer Destillationsanlage, ist es möglich, eventuell anfallende hochsiedende Komponenten der Mischung, die als Nebenprodukte aus der Umsetzung der organischen Verbindung mit dem Hydroperoxid anfallen, über Sumpf abzutrennen. Dabei ist es auch denkbar, beispielsweise durch Zugabe von vorzugsweise gasförmigen, niedrigsiedenden Komponenten, wie z.B. der organischen Verbindung, vorzugsweise Propen, an sich, die Sumpftemperatur zu erniedrigen.

Beispiele für solche niedrigsiedenden Komponenten sind u.a. Kohlenwasserstoffe mit 1 bis 4 Kohlenstoffatomen wie beispielsweise Methan, Ethan, Propan, Butan, Ethen oder Butene. Ebenso können beispielsweise Stickstoff oder Argon eingesetzt werden.

Selbstverständlich ist es möglich, auch mehrere organische Verbindungen mit dem Hydroperoxid umzusetzen. Ebenso ist es denkbar, zur Umsetzung mehrere Hydroperoxide zu verwenden.

Werden mehrere organische Verbindungen und/oder mehrere Hydroperoxide miteinander in den jeweiligen Stufen umgesetzt, so können in den Mischungen verschiedenartige Produkte, die aus den Umsetzungen resultieren, vorliegen. Werden diese wiederum in den jeweiligen Abtrennstufen destillativ abgetrennt, kann es notwendig sein, zur Abtrennung mehrer Destillationskolonnen vorzusehen. Ebenso kann die destillative Abtrennung mehrerer Hydroperoxide aus der Mischung mehrere Destillationskolonnen erforderlich machen.

Die Umsetzung der organischen Verbindung mit dem Hydroperoxid in der Stufe (i) findet in einem dafür geeigneten Reaktor R_{I} statt. Als Edukte der Umsetzung werden die umzusetzende organische Verbindung, das Hydroperoxid und, sofern erforderlich, ein oder auch mehrere bei der Umsetzung geeignete und/oder erforderliche Lösungsmittel eingesetzt.

In den Reaktor R_{I} fließen also mindestens die Ströme E_{I}¹ und E_{I}². Gegebenenfalls kann beispielsweise ein weiterer Strom E_{I}³ in den Reaktor R_{I} fließen. Dabei bezeichnet
E_{I}¹ den Strom, der die umzusetzende Verbindung enthält, gegebenenfalls gelöst in einem oder mehreren Lösungsmitteln,
E_{I}² den Strom, der das Hydroperoxid enthält, gegebenenfalls gelöst in einem oder mehreren Lösungsmitteln, und
E_{I}³ den Strom, der ein oder mehrere Lösungsmittel enthält.

Die einzelnen Ströme E_{I}¹ werden vor dem Zufluß in den Reaktor R_{I} vorzugsweise zu einem Strom E_{I} vereinigt. Ebenso ist es prinzipiell möglich, die einzelnen Ströme einzeln in den Reaktor R_{I} zu leiten. Weiter ist es auch möglich, die einzelnen Ströme, in sinnvollen Kombinationen zusammengeführt, in den Reaktor R_{I} zu leiten. Beispielsweise könnten E_{I}¹ und E_{I}³ vor dem Eingang in den Reaktor R_{I} zusammengeführt und in den Reaktor R_{I} geleitet werden, in den als separater Strom zusätzlich der Strom E_{I}² fließt.

Bevorzugt wird ein Strom E_{I} in den Reaktor R_{I} geleitet, der aus der Kombination der Ströme E_{I}¹, E_{I}² und E_{I}³ besteht. Dabei ist ein Strom bevorzugt, bei dem die Konzentrationen der einzelnen Komponenten des Stroms so gewählt sind, daß der Strom flüssig und einphasig ist.

Bevorzugt werden dabei Hydroperoxidkonzentrationen in E_{I} verwendet, die im Bereich von 0,01 bis 10, besonders bevorzugt im Bereich von 0,1 bis 9 Gew.-%, weiter besonders bevorzugt im Bereich von 1 bis 8 Gew.-% und insbesondere im Bereich von 5 bis 7 Gew.-% liegen.

Die Konzentration der umzusetzenden organischen Verbindung wird beispielsweise so gewählt, daß das molare Verhältnis von umzusetzender organischer Verbindung zu Hydroperoxid im Bereich von 0,7 bis 3,0, bevorzugt im Bereich von 0,8 bis 2,7, weiter besonders bevorzugt im Bereich von 0,9 bis 2,3 und insbesondere im Bereich von 1,0 bis 2,0 liegt.

Je nach Temperatur, die zur Umsetzung der organischen Verbindung mit dem Hydroperoxid im Reaktor R_{I} gewählt wird, kann es sinnvoll sein, den Strom oder die Ströme vor dem Eingang in den Reaktor R_{I} vorzutemperieren.

Die Reaktionsbedingungen werden im Reaktor R_{I} so gewählt, daß der Hydroperoxidumsatz im allgemeinen im Bereich von 70 bis 95 %, bevorzugt im Bereich von 80 bis 94,5 %, und insbesondere bevorzugt im Bereich von 85 bis 94 % liegt.

Weiter werden Druck p_{I}, Temperatur T_{I} und Verweilzeit Δt_{I} des Reaktionsgutes im Reaktor R_{I} bevorzugt so gewählt, daß die Mischung M_{I}, die aus der Umsetzung resultiert, flüssig und einphasig ist.

Dabei werden Drücke p_{I} gewählt, die im allgemeinen im Bereich vom Eigendruck bis 100 bar liegen, bevorzugt im Bereich vom Eigendruck bis 40 bar und besonders bevorzugt im Bereich vom Eigendruck bis 30 bar.

Die Temperaturen T_{I} liegen im allgemeinen im Bereich von 0 bis 120 °C, bevorzugt im Bereich von 10 bis 100 °C, weiter bevorzugt im Bereich von 20 bis 90 °C und besonders bevorzugt im Bereich von 30 bis 80 °C.

Nach der Umsetzung im Reaktor R_{I} wird die resultierende Mischung als Strom M_{I} der Abtrennvorrichtung A_{I} zugeführt. Dort wird, wie oben beschrieben, das Hydroperoxid aus der Mischung abgetrennt.

Erfolgt bei destillativer Abtrennung hierbei auch die Abtrennung von umgesetzter organischer Verbindung, so wird die Destillation im allgemeinen so geführt, daß aus M_{I} mindestens 50 %, bevorzugt mindestens 60 %, weiter bevorzugt mindestens 70 %, besonders bevorzugt mindestens 80 % und insbesondere bevorzugt mindestens 90 % der umgesetzten organischen Verbindung abgetrennt werden.

Bevorzugt wird die Abtrennung so geführt, daß eine flüssige Mischung, die das Hydroperoxid enthält, abgetrennt wird. Diese abgetrennte Mischung wird im folgenden mit M_{I}² bezeichnet. Dabei ist es möglich, daß die abgetrennte Mischung, die das Hydroperoxid enthält, zusätzlich zum Hydroperoxid beispielsweise noch geringe Mengen an nicht umgesetzter organischer Verbindung und/oder umgesetzter organischer Verbindung enthält. Ebenso kann die Mischung M_{I}², die das abgetrennte Hydroperoxid enthält, gegebenenfalls erforderliches Lösungsmittel enthalten, das über den Strom E_{I}³ zugesetzt wurde, oder Lösungsmittel, das gegebenenfalls in den Strömen E_{I}¹ und/oder E_{I}² enthalten war.

Wird in der Abtrennvorrichtung A_{I} auch die umgesetzte organische Verbindung abgetrennt, so resultiert aus dieser Abtrennung, aus der bevorzugt eine flüssige Mischung oder eine Flüssigkeit-Gas-Mischung erhalten wird, ein Strom, der im folgenden mit M_{I}¹ bezeichnet wird. Dieser enthält neben der umgesetzten organischen Verbindung gegebenenfalls die nicht umgesetzte organische Verbindung und/oder geringe Mengen an gegebenenfalls erforderlichem Lösungsmittel, das über den Strom E_{I}³ zugesetzt wurde, oder Lösungsmittel, das gegebenenfalls in den Strömen E_{I}¹ und/oder E_{I}² enthalten war.

Wird, wie oben beschrieben, die Abtrennung in einer Destillationsanlage durchgeführt, und werden über Sumpf hochsiedende Anteile von M_{I} abgetrennt, so resultiert aus dieser Abtrennung ein Strom M_{I}³. Solche hochsiedenden Anteile können beispielsweise Nebenprodukte der Umsetzung in Reaktor R_{I} sein, die im Strom M_{I} enthalten sind.

Nach dem Durchlauf der Stufen (i) und (ii) wird das abgetrennte Hydroperoxid in der Stufe (iii) erneut mit der organischen Verbindung umgesetzt.

Der Strom M₁² wird in einen zweiten Reaktor R_{II} geleitet. Der Strom M_{I}² stellt also, bezogen auf den Reaktor R_{II}, in Analogie zu den Strömen, die in den Reaktor R_{I} fließen, den Strom E_{II}² dar. Da im Reaktor R_{II} gemäß Stufe (iü) eine erneute Umsetzung des abgetrennten Hydroperoxides mit der umzusetzenden organischen Verbindung stattfindet, ist bezüglich des Reaktors R_{II} mindestens ein weiterer Strom E_{II}¹ erforderlich. Gegebenenfalls kann beispielsweise auch ein Strom E_{II}³ erforderlich sein.

Dabei bezeichnet, analog zu den oben beschriebenen Strömen E_{I}¹ bis E_{I}³ ,
- E_{II}¹: den Strom, der die umzusetzende Verbindung enthält, gegebenenfalls gelöst in einem oder mehreren Lösungsmitteln,
- E_{II}²: den Strom, der das Hydroperoxid enthält, gegebenenfalls gelöst in einem oder mehreren Lösungsmitteln, und
- E_{II}³: den Strom, der ein oder mehrere Lösungsmittel enthält.

Ebenfalls analog zu den oben beschriebenen Strömen E_{I}¹ ist es möglich, die Ströme E_{II}ⁱ einzeln oder zusammengeführt in geeigneten Kombinationen in den Reaktor R_{II} zu leiten. Ebenso ist, wie oben beschrieben, eine Vortemperierung der Ströme E_{II}ⁱ möglich.

Vorzugsweise wird der Strom E_{II}² mit einem Strom E_{II}¹ oder einem Strom E_{II}¹ + E_{II}³ zusammengeführt und der resultierende Strom in R_{II} geleitet. Die Konzentrationen der Komponenten der Ströme E_{II}¹ bzw. E_{II}³ werden dabei bevorzugt so gewählt, daß der Strom E_{II} , der in den Reaktor R_{II} fließt, flüssig und einphasig ist.

Dabei wird die Konzentration der umzusetzenden organischen Verbindung so gewählt, daß das molare Verhältnis von umzusetzender organischer Verbindung zu Hydroperoxid bevorzugt im Bereich von 0,7 bis 10,0, weiter bevorzugt im Bereich von 0,8 bis 8,0, besonders bevorzugt im Bereich von 0,9 bis 6,0 und insbesondere im Bereich von 1,0 bis 4,0 liegt.

Wie im Reaktor R_{I} wird die Umsetzung im Reaktor R_{II} bei einem Druck p_{II}, einer Temperatur T_{II} und einer Verweilzeit Δt_{II} des Reaktionsgutes durchgeführt, daß Hydroperoxidumsätze erzielt werden, die im allgemeinen im Bereich von ≥ 90%, bevorzugt im Bereich von ≥ 92%, weiter bevorzugt im Bereich ≥ 95% und besonders bevorzugt im Bereich von 95 bis 99,5 % liegen.

Dabei werden Drücke p_{II} gewählt, die im allgemeinen im Bereich vom Eigendruck bis 100 bar liegen, bevorzugt im Bereich vom Eigendruck bis 40 bar und besonders bevorzugt im Bereich vom Eigendruck bis 30 bar.

Die Temperaturen T_{II} liegen im allgemeinen im Bereich von 0 bis 120 °C, bevorzugt im Bereich von 10 bis 100 °C, weiter bevorzugt im Bereich von 20 bis 90 °C und besonders bevorzugt im Bereich von 30 bis 80 °C.

Selbstverständlich ist es möglich, die Mischung M_{II}, die aus der Umsetzung im Reaktor R_{II} resultiert, dem Reaktor R_{II} zu entnehmen und, wie oben bereits beschrieben, einer Abtrennvorrichtung A_{II} oder auch der Abtrennvorrichtung A_{I} zuzuführen und gegebenenfalls eine dritte Umsetzung anzuschließen.

In einer bevorzugten Ausführungsform werden jedoch zwei Reaktoren R_{I} und R_{II} sowie eine Abtrennvorrichtung A_{I} verwendet.

Als Reaktoren können selbstverständlich alle denkbaren, für die jeweiligen Reaktionen am besten geeigneten Reaktoren eingesetzt werden. Dabei ist ein Reaktor nicht auf einen einzelnen Behälter beschränkt. Vielmehr ist es auch möglich, als beispielsweise Reaktor R_{I} oder beispielsweise Reaktor R_{II} eine Rührkesselkaskade einzusetzen.

Als Reaktoren werden Festbettreaktoren verwendet. Weiter bevorzugt werden als Festbettreaktoren Festbettrohrreaktoren eingesetzt.

Insbesondere wird als Reaktor R_{I} ein isothermer Festbettreaktor und als Reaktor R_{II} ein adiabatischer Festbettreaktor verwendet.

Daher betrifft die vorliegende Erfindung auch eine Vorrichtung, umfassend einen isothermen Festbettreaktor (I), eine Abtrenneinrichtung (II) und einen adiabatischen Festbettreaktor (III).

Ebenso betrifft die vorliegende Erfindung die Verwendung dieser Vorrichtung zur Umsetzung einer organischen Verbindung mit einem Hydroperoxid.

Weiter betrifft die vorliegende Erfindung diese Verwendung, wobei zur Umsetzung der organischen Verbindung mit dem Hydroperoxid die folgenden Stufen (i) bis (iii) durchlaufen werden:
(i) Umsetzung des Hydroperoxides mit der organischen Verbindung unter Erhalt einer Mischung, umfassend die umgesetzte organische Verbindung und nicht umgesetztes Hydroperoxid,
(ü) Abtrennung des nicht umgesetzten Hydroperoxides aus der aus Stufe (i) resultierenden Mischung,
(iii) Umsetzung des abgetrennten Hydroperoxides aus Stufe (ü) mit der organischen Verbindung.

Als Hydroperoxid wird Wasserstoffperoxid eingesetzt. Bevorzugt wird dabei eine wäßrige Wasserstoffperoxidlösung verwendet.

Zur Herstellung von Wasserstoffperoxid kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmanns Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.
Möglich ist selbstverständlich auch die Darstellung von Wasserstoffperoxid aus den Elementen.

Im verfahren werden zur größeren Effizienz der Umsetzung ein oder mehrere geeignete heterogene Katalysatoren zugesetzt.

Dabei sind prinzipiell alle heterogenen Katalysatoren denkbar, die für die jeweilige Umsetzung geeignet sind. Bevorzugt werden dabei Katalysatoren verwendet, die ein poröses oxidisches Material wie z.B. ein Zeolith umfassen. Vorzugsweise werden Katalysatoren eingesetzt, die als poröses oxidisches Material ein Titan-, Vanadium-, Chrom-, Niob- oder Zirkoniumhaltigen Zeolith umfassen.

Dabei sind im einzelnen Titan-, Vanadium-, Chrom-, Niob-, Zirkoniumhaltige Zeolithe mit Pentasil-Zeohth-Siruktur, insbesondere die Typen mit röntgenografischer Zuordnung zur BEA-, MOR-, TON-, MTW-, FER-, MFI-, MEL-, CHA-, ERI-, RHO-, GIS-, BOG-, NON-, EMT-, HEU-, KFI-, FAU-, DDR-, MTT-, RUT-, RTH-, LTL-, MAZ-, GME-, NES-, OFF-, SGT-, EUO-, MFS-, MWW- oder MFI/MEL-Mischstruktur sowie ITQ-4 zu nennen. Denkbar sind für den Einsatz im erfindungsgemäßen Verfahren weiterhin titanhaltige Zeolithe mit der Struktur des UTD-1, CIT-1 oder CIT-5. Als weitere titanhaltige Zeolithe sind solche mit der Struktur des ZSM-48 oder ZSM-I2 zu nennen.
Als besonders bevorzugt sind Ti-Zeolithe mit MFI-, MEL- oder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Insbesondere bevorzugt wird ein heterogener Katalysator, der das titanhaltige Silikalit TS-1 umfaßt, verwendet.

Dabei ist es möglich, als Katalysator das poröse oxidische Material an sich zu verwenden. Selbstverständlich ist es jedoch auch möglich, als Katalysator einen Formkörper einzusetzen, der das poröse oxidische Material umfaßt. Dabei können zur Herstellung des Formkörpers, ausgehend von dem porösen oxidischen Material, alle Verfahren gemäß dem Stand der Technik eingesetzt werden.

Vor, während oder nach dem einen oder mehreren Formgebungsschritten in diesen Verfahren können auf das Katalysatormaterial Edelmetalle in Form geeigneter Edelmetallkomponenten, beispielsweise in Form von wasserlöslichen Salzen aufgebracht werden. Vorzugsweise wird dieses Verfahren angewendet, um Oxidationskatalysatoren auf der Basis von Titan- oder Vanadiumsilikaten mit Zeolithstruktur herzustellen, wobei Katalysatoren erhältlich sind, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen. Derartige Katalysatoren sind beispielsweise in der DE-A 196 23 609.6 beschrieben, die hiermit bzgl. der darin beschriebenen Katalysatoren voll umfänglich in den Kontext der vorliegenden Anmeldung durch Bezugnahme aufgenommen wird.

Selbstverständlich können die Formkörper konfektioniert werden. Sämtliche Verfahren zur Zerkleinerung sind dabei denkbar, beispielsweise durch Splittung oder Brechen der Formkörper, ebenso wie weitere chemische Behandlungen, wie beispielsweise vorstehend beschrieben.

Bei Verwendung eines Formkörpers oder auch mehr davon als Katalysator kann dieser nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff-liefernden Substanzen enthält. Dieses Regenerierungsverfahren ist in der DE-A 197 23 949.8 beschrieben.

Unter den Reaktionen, die im Verfahren möglich sind, seien beispielhaft die folgenden genannt:
die Epoxidation von Olefinen wie z.B. die Herstellung von Propenoxid aus Propen und H₂O₂ oder aus Propen und Gemischen, die H₂O₂ in situ liefern;

Hydroxylierungen wie z.B die Hydroxylierung mono-, bi- oder polycyclischer Aromaten zu mono-, di- oder höher substituierten Hydroxyaromaten, beispielsweise die Umsetzung von Phenol und H₂O₂ oder von Phenol und Gemischen, die H₂O₂ in situ liefern, zu Hydrochinon; die Oximbildung aus Ketonen unter Anwesenheit von H₂O₂ oder Gemischen, die H₂O₂ in situ liefern, und Ammoniak (Ammonoximierung), beispielsweise die Herstellung von Cyclohexanonoxim aus Cyclohexanon;
die Baeyer-Villiger-Oxidation.

Bevorzugt werden organische Verbindungen umgesetzt, die mindestens eine C-C-Doppelbindung aufweisen. Demgemäß betrifft die vorliegende Erfindung ein Verfahren, wie oben beschrieben, das dadurch gekennzeichnet ist, daß die organische Verbindung mindestens eine C-C-Doppelbindung aufweist.

Als Beispiele für solche organischen Verbindungen mit mindestens einer C-C-Doppelbindung seien folgende Alkene genannt:
Ethen, Propen, 1-Buten, 2-Buten, Isobuten, Butadien, Pentene, Piperylen, Hexene, Hexadiene, Heptene, Octene, Diisobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbomen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Bevorzugt werden Alkene verwendet, die 2 bis 8 Kohlenstoffatome enthalten. Besonders bevorzugt werden Ethen, Propen, und Buten umgesetzt. Insbesondere bevorzugt wird Propen umgesetzt.

Ein weiterer Vorteil des Verfahrens, neben der Tatsache, daß ein geringerer Überschuß an umzusetzender organischer Verbindung im Verhältnis zu Hydroperoxid erreicht werden kann, ist darin zu sehen, daß über die Abtrennung von Hydroperoxid und dessen erneute Umsetzung mit der organischen Verbindung ein hoher Gesamtumsatz des Hydroperoxides erzielt werden kann. Gleichzeitig werden Folgereaktionen des Produktes reduziert.

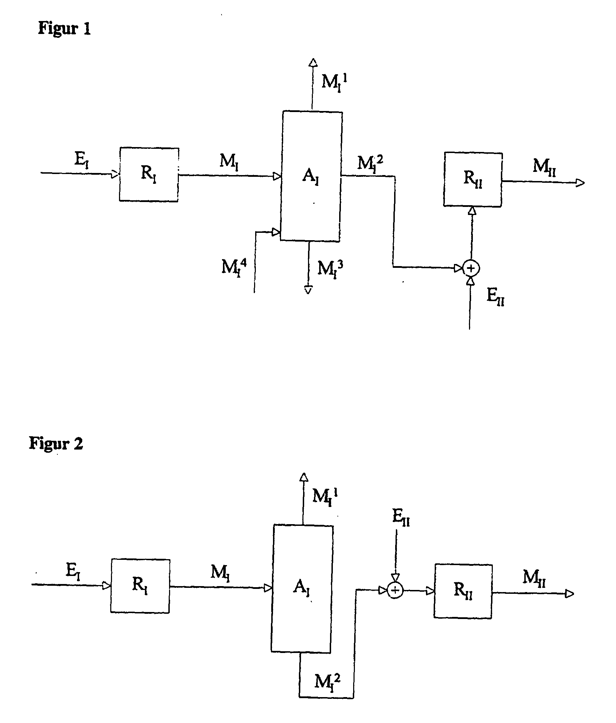

In Figur 1 ist eine bevorzugte Ausführungsform der Apparatur dargestellt. Dabei bezeichnet
- E_{I}: einen Strom, enthaltend beispielsweise flüssiges Propen, wäßrige Wasserstoffperoxidlösung und Methanol,
- R_{I}: einen isothermen Festbettrohrreaktor,
- M_{I}: einen Strom, resultierend aus der Umsetzung in Reaktor R_{I},
- A_{I}: eine Destillationskolonne zur Abtrennung über Kopf, über Seitenabzug und über Sumpf,
- M_{I}¹: einen Strom, resultierend aus der Abtrennung über Kopf, der vorwiegend Propen, Propenoxid und Methanol umfaßt,
- M_{I}²: einen Strom, resultierend aus der Abtrennung über Seitenabzug, der vorwiegend Methanol und wäßrige Wasserstoffperoxidlösung umfaßt und der in den Reaktor R_{II} geleitet wird,
- M_{I}³: einen Strom aus der Abtrennung über Sumpf, der hochsiedende Nebenprodukte, beispielsweise Methoxypropanole und Propantriol, aus der Umsetzung in Reaktor R_{I} umfaßt,
- M_{I}⁴: einen optionalen Strom, der der Destillationsanlage A_{I} zugegeben wird, um die Sumpftemperatur niedrig zu halten, beispielsweise gasförmiges Propen,
- R_{II}: einen adiabatischen Festbettrohrreaktor,
- E_{II}: einen Strom, der flüssiges Propen und Methanol umfaßt und in den Reaktor R_{II} geleitet wird,
- M_{II}: einen Strom aus Reaktor R_{II}, der Propen, Propenoxid und Methanol umfaßt.

In Figur 2 ist eine weitere bevorzugte Ausführungsform der Apparatur dargestellt. Dabei bezeichnet
- E_{I}: einen Strom, enthaltend beispielsweise flüssiges Propen, wäßrige Wasserstoffperoxidlösung und Methanol,
- R_{I}: einen isothermen Festbettrohrreaktor,
- M_{I}: einen Strom, resultierend aus der Umsetzung in Reaktor R_{I},
- A_{I}: eine Destillationskolonne zur Abtrennung über Kopf und über Sumpf,
- M_{I}¹: einen Strom, resultierend aus der Abtrennung über Kopf, der vorwiegend Propen, Propenoxid und Methanol umfaßt,
- M_{I}²: einen Strom, resultierend aus der Abtrennung über Sumpf, der vorwiegend Wasserstoffperoxid, Wasser, Methanol und hochsiedende Nebenprodukte umfaßt und der in den Reaktor R_{II} geleitet wird,
- R_{II}: einen adiabatischen Festbettrohrreaktor,
- E_{II}: einen Strom, der flüssiges Propen und Methanol umfaßt und in den Reaktor R_{II} geleitet wird,
- M_{II}: einen Strom aus Reaktor R_{II}, der Propen, Propenoxid und Methanol umfaßt.

### Beispiele

### Beispiel 1: Zweistufige Fahrweise mit Zwischenabtrennung

Durch einen ersten Rohrreaktor mit ca. 50 ml Reaktionsvolumen, gefüllt mit 23.1 g verstrangtem TS-1, wurden Flüsse von 10,5 g/h Wasserstoffperoxid (ca. 40 Gew.-%), 58 g/h Methanol und 10 g/h Propen bei 40 °C Reaktionstemperatur und 20 bar Reaktionsdruck geleitet.

Zur Analyse des Austrags des Rohrreaktors wurde die Reaktionsmischung in einen Sambay-Verdampfer gegen Atmosphärendruck entspannt. Die abgetrennten Leichtsieder wurden online in einem Gaschromatographen analysiert. Der flüssige Reaktionsaustrag wurde gesammelt, gewogen und ebenfalls gaschromatographisch analysiert.

Der erreichte Wasserstoffperoxid-Umsatz betrug 85 %. Die Propenoxid-Selektivität bezüglich Wasserstoffperoxid betrug 95 %.

Der Austrag aus dem ersten Reaktor, der Methanol, Wasser, Propenoxid, Nebenprodukte, unumgesetztes Propen und Wasserstoffperoxid enthielt, wurde in eine Kolonne entspannt. Die Kolonne wurde bei Normaldruck betrieben und hatte ca. 15 theoretische Stufen.

Bei einer Sumpftemperatur von ca. 69 °C gelang eine Abtrennung des Propenoxids aus der Mischung bis auf < 1 Gew.-%.

Über Kopf gingen hierbei neben Propenoxid das leichter siedende Propen und Teile des Methanols. Am Kopf wurde bei 50 °C in einem Teilkondensator der für die Trennung in der Kolonne benötigte Rücklauf kondensiert. Das Kopfprodukt wurde gasförmig abgezogen und der Aufarbeitung zugeführt.

Das Sumpfprodukt wurde einem zweiten Rohrreaktor zugeführt.

Durch einen zweiten Rohrreaktor mit ca. 50 ml Reaktionsvolumen, gefüllt mit 28 g verstrangtem TS-1, wurden das Sumpfprodukt aus der Zwischenabtrennung und ein Propenstrom von ca. 9 g/h bei 40 °C Reaktionstemperatur und 20 bar Reaktionsdruck geleitet.
Nach Verlassen des Reaktors wurde die Reaktionsmischung in einem Sambay-Verdampfer gegen Atmosphärendruck entspannt. Die abgetrennten Leichtsieder wurden online in einem Gaschromatographen analysiert. Der flüssige Reaktionsaustrag wurde gesammelt, gewogen und ebenfalls gaschromatographisch analysiert.

Der erreichte Wasserstoffperoxid-Umsatz betrug 96 %. Die Propenoxid-Selektivität bezüglich Wasserstoffperoxid betrug 96 %.

In der Summe betrug der Wasserstoffperoxid-Umsatz 99,4 % und die Propenoxid-Selektivität 95-96 %. Dies ergab eine Propenoxid-Ausbeute bezüglich Wasserstoffperoxid von 94-95 %.

### Beispiel 2: Einstufige Fahrweise ohne Zwischenabtrennung

Durch einen Rohrreaktor mit ca. 50 ml Reaktionsvolumen, gefüllt mit 20 g verstrangtem TS-1, wurden Flüsse von 8,3 g/h Wasserstoffperoxid (ca. 40 Gew.-%), 49 g/h Methanol und 7,8 g/h Propen bei 40 °C Reaktionstemperatur und 20 bar Reaktionsdruck geleitet.

Nach Verlassen des Reaktors wurde die Reaktionsmischung in einen Sambay-Verdampfer gegen Atmosphärendruck entspannt. Die abgetrennten Leichtsieder wurden online in einem Gaschromatographen analysiert. Der flüssige Reaktionsaustrag wurde gesammelt, gewogen und ebenfalls gaschromatographisch analysiert.

Der erreichte Wasserstoffperoxid-Umsatz betrug 98,4 %. Die Propenoxid-Selektivität bezüglich Wasserstoffperoxid betrug 80,3 %. Die Propenoxid-Ausbeute bezüglich Wasserstoffperoxid betrug 79 %.

## Patentansprüche

1. Verfahren zur Umsetzung einer organischen Verbindung mit einem Hydroperoxid, das mindestens die folgenden Stufen (i) bis (iii) umfasst:
(i) Umsetzung des Hydroperoxides mit der organischen Verbindung unter Erhalt einer Mischung, umfassend die umgesetzte organische Verbindung und nicht umgesetztes Hydroperoxid,
(ii) Abtrennung des nicht umgesetzten Hydroperoxides aus der aus Stufe (i) resultierenden Mischung,
(iii) Umsetzung des abgetrennten Hydroperoxides aus Stufe (ii) mit der organischen Verbindung,
**dadurch gekennzeichnet, dass** als Hydroperoxid Wasserstoffperoxid verwendet wird, die organische Verbindung bei der Umsetzung mit einem heterogenen Katalysator in Kontakt gebracht wird und die Umsetzungen in den Stufen (i) und (iii) in zwei getrennten Festbettreaktoren durchgeführt werden.

2. Verfahren nach Anspruch 1, **dadurch gekennzeichnet, dass** der heterogene Katalysator ein titanhaltiges Silicalit umfasst.

3. Verfahren nach einem der Ansprüche 1 bis 2, **dadurch gekennzeichnet, dass** die organische Verbindung mindestens eine C-C-Doppelbindung aufweist.

4. Vorrichtung, umfassend einen isothermen Festbettreaktor (I), eine Abtrenneinrichtung (II) und einen adiabatischen Festbettreaktor (III).

5. Verwendung einer Vorrichtung gemäß Anspruch 4 zur Umsetzung einer organischen Verbindung mit einem Hydroperoxid.

6. Verwendung nach Anspruch 5, wobei zur Umsetzung der organischen Verbindung mit dem Hydroperoxid die folgenden Stufen (i) bis (iii) durchlaufen werden:
(i) Umsetzung des Hydroperoxides mit der organischen Verbindung unter Erhalt einer Mischung, umfassend die umgesetzte organische Verbindung und nicht umgesetztes Hydroperoxid,
(ii) Abtrennung des nicht umgesetzten Hydroperoxides aus der aus Stufe (i) resultierenden Mischung,
(iii) Umsetzung des abgetrennten Hydroperoxides aus Stufe (ii) mit der organischen Verbindung.

7. Verwendung nach Anspruch 6, **dadurch gekennzeichnet, dass** die Umsetzung in Stufe (i) in einem isothermen Festbettreaktor (I), die Abtrennung in Stufe (ii) mittels einer Abtrenneinrichtung (II) und die Umsetzung in Stufe (iii) in einem adiabatischen Festbettreaktor (III) durchgeführt wird.

## Claims

1. A process for the reaction of an organic compound with a hydroperoxide, which comprises at least the steps (i) to (iii) below:
(i) reaction of the hydroperoxide with the organic compound to give a mixture comprising the reacted organic compound and unreacted hydroperoxide,
(ii) separation of the unreacted hydroperoxide from the mixture resulting from step (i),
(iii) reaction of the hydroperoxide separated off in step (ii) with the organic compound, wherein the hydroperoxide used is hydrogen peroxide, the organic compound is brought into contact with a heterogeneous catalyst during the reaction and the reactions in steps (i) and (iii) are carried out in two separate fixed-bed reactors.

2. A process as claimed in claim 1, wherein the heterogeneous catalyst comprises a titanium-containing silicalite.

3. A process as claimed in either of claims 1 and 2, wherein the organic compound has at least one C-C double bond.

4. An apparatus comprising an isothermal fixed-bed reactor (I), a separation apparatus (II) and an adiabatic fixed-bed reactor (III).

5. The use of an apparatus as claimed in claim 4 for the reaction of an organic compound with a hydroperoxide.

6. The use as claimed in claim 5, wherein the steps (i) to (iii) below:
(i) reaction of the hydroperoxide with the organic compound to give a mixture comprising the reacted organic compound and unreacted hydroperoxide,
(ii) separation of the unreacted hydroperoxide from the mixture resulting from step (i),
(iii) reaction of the hydroperoxide separated off in step (ii) with the organic compound,
are carried out for the reaction of the organic compound with the hydroperoxide.

7. The use as claimed in claim 6, wherein the reaction in step (i) is carried out in an isothermal fixed-bed reactor (I), the separation in step (ii) is carried out by means of a separation apparatus (II) and the reaction in step (iii) is carried out in an adiabatic fixed-bed reactor (III).

## Revendications

1. Procédé pour la mise en réaction d'un composé organique avec un hydroperoxyde, qui comprend au moins les étapes (i) à (iii) ci-après consistant à :
(i) faire réagir l'hydroperoxyde avec le composé organique pour obtenir un mélange comprenant le composé organique ayant réagi et de l'hydroperoxyde n'ayant pas réagi ;
(ii) séparer l'hydroperoxyde n'ayant pas réagi du mélange résultant de l'étape (i) ;
(iii) faire réagir l'hydroperoxyde séparé de l'étape (ii) avec le composé organique
**caractérisé en ce qu'**on utilise, à titre d'hydroperoxyde, du peroxyde d'hydrogène, on amène le composé organique, lors de la mise en réaction, en contact avec un catalyseur hétérogène et on effectue les mises en réaction aux étapes (i) et (iii) dans deux réacteurs séparés du type à lit fixe.

2. Procédé selon la revendication 1, **caractérisé en ce que** le catalyseur hétérogène comprend une silicalite contenant du titane.

3. Procédé selon l'une quelconque des revendications 1 à 2, **caractérisé en ce que** le composé organique présente au moins une liaison double carbone-carbone.

4. Dispositif, comprenant un réacteur isothermique du type à lit fixe (I), un dispositif de séparation (II) et un réacteur adiabatique du type à lit fixe (III).

5. Utilisation d'un dispositif selon la revendication 4, pour la mise en réaction d'un composé organique avec un hydroperoxyde.

6. Utilisation selon la revendication 5, dans laquelle, pour la mise en réaction du composé organique avec l'hydroperoxyde, on passe par les étapes (i) à (iii) ci-après consistant à :
(i) faire réagir l'hydroperoxyde avec le composé organique pour obtenir un mélange comprenant le composé organique ayant réagi et de l'hydroperoxyde n'ayant pas réagi ;
(ii) séparer l'hydroperoxyde n'ayant pas réagi du mélange résultant de l'étape (i) ;
(iii) faire réagir l'hydroperoxyde séparé de l'étape (ii) avec le composé organique.

7. Utilisation selon la revendication 6, **caractérisé en ce que** la mise en réaction à l'étape (i) a lieu dans un réacteur isothermique du type à lit fixe (I), la séparation à l'étape (ii) a lieu à l'aide d'un dispositif de séparation (II) et la mise en réaction à l'étape (iii) a lieu dans un réacteur adiabatique du type à lit fixe (III).
